# EUROPEAN PATENT APPLICATION

(11) **EP 3 964 137 A1**
(43) Date of publication of application: **09.03.2022**
(21) Application number: 20194483.2
(22) Date of filing: 04.09.2020
(51) Int. Cl.: A61B 8/12, A61B 5/05, A61B 8/00, A61B 8/08

(54) **MAGNETOMOTIVE PROBE**

(71) Applicant: NanoEcho AB, 222 29 Lund (SE)
(72) Inventor: Evertsson, Maria, 224 80 Lund (SE); Jansson, Tomas, 224 65 Lund (SE)
(74) Representative: KIPA AB

(57) **Abstract**

A magnetomotive imaging probe device is described. The probe device includes a housing having an outer surface and an inner cavity; a magnet; a sensing device configured to detect distance, movement, or magnetic material. The magnet is arranged in the inner cavity of the housing and the sensing device on the outer surface of the housing, and the magnet is arranged to generate a time-varying magnetic field at an imaging plane of the sensing device. The magnet is intended to move magnetic nanoparticles in tissue such that a movement can be detected with the sensing device (ultrasound, optical or other). The detected motion infers the presence of magnetic material (nanoparticles).

## Description

### Field of the Invention

This invention pertains in general to the field of magnetomotive imaging. More particularly the invention relates to a magnetomotive imaging probe device. The magnetomotive imaging probe device is designed so that it could be to be arranged in a cavity of an animal or human, such as for transrectal or transvaginal use, but the device may also be designed or utilized for external use.

### Background of the Invention

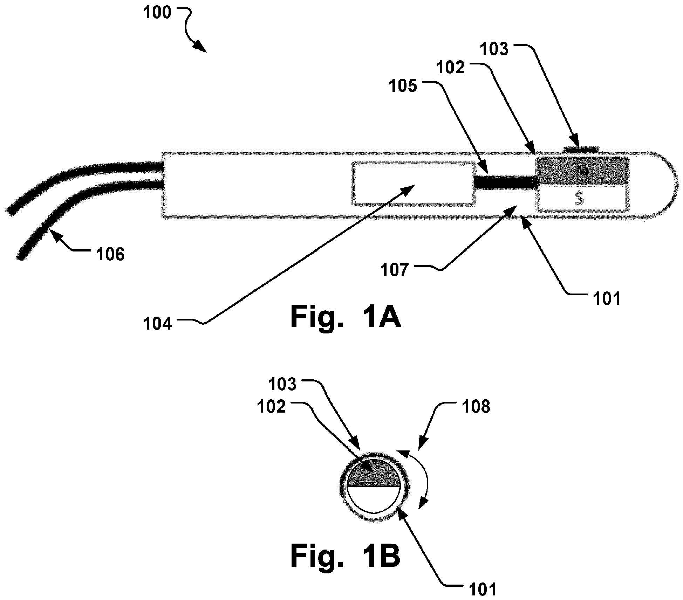

Magnetomotive imaging is an imaging technique where superparamagnetic iron oxide nanoparticles can be used as ultrasound contrast agents. The main idea of this imaging technique is the application of a time-varying magnetic field (pulsed or sinusoidal) to the volume where the nanoparticles are deposited. The magnetic field induces movement of the particles and thereby the surrounding tissue, and the movement is detected with ultrasound. In early implementations, such as disclosed in Evertsson, M. et al, IEEE, Transactions on ultrasonic, ferroelectrics, and frequency control, vol. 60, no. 3, 1 March 2013, pages 481-491, an electromagnet was employed to create the time-varying magnetic field, the electromagnet consisting of a coil around a cone-shaped iron-core, see Fig. 1. When a current is applied, a magnetic field is formed from the tip of the core. The force acting on the particles is dependent on the field strength, and on the field gradient.

Several problems exist with the electromagnet approach, in that the magnets tend to be heavy and demand high currents to produce a sufficient magnetic field, the later fact causing substantial heat. These facts make it difficult to produce magnetomotive systems that can be used clinically, especially in systems aimed for endoscopical applications. A magnetic field generator was proposed in EP 2801323, where a permanent magnet was used instead and that this magnet was rotated in such a way that north and south poles were alternately pointing towards the region where superparamagnetic nanoparticles had collected, and to that effect, exhibiting the particles to a varying magnetic force, attracting them alternately to the magnet.

The rotating permanent magnet solution was suggested as a magnetic field generator and separated from a standard ultrasound transducer connected to an ultrasound scanner. The device provided limited use due to its design and the limited range of the magnetic force.

Hence, a more user-friendly, compact and versatile magnetomotive imaging probe device, method and/or system which could be used at more locations on a human and/or animal would be an advantageous. Further improved sensitivity of detection of magnetic nanoparticles would also be an advantage together with improved patient safety, more cost-effective diagnosis.

### Summary of the Invention

Accordingly, examples of the present invention preferably seek to mitigate, alleviate or eliminate one or more deficiencies, disadvantages or issues in the art, such as the above-identified, singly or in any combination by providing a magnetomotive imaging probe assembly and a method for magnetomotive imaging with a probe assembly according to the appended patent claims.

According to a first aspect of the disclosure a magnetomotive imaging probe system is provided. The magnetomotive imaging probe device may comprise a housing having an outer surface and an inner cavity; a magnet; a sensing device configured to detect distance, movement, or magnetic material. The magnet may be arranged in the inner cavity of the housing and the sensing device may be arranged on the outer surface of the housing. The magnet may be arranged to generate a time-varying magnetic field at an imaging plane of the sensing device.

In some examples of the magnetomotive imaging probe, the sensing device may be an ultrasound transducer.

In some examples of the magnetomotive imaging probe, the magnet may be a cylindrically shaped permanent magnet and the time-varying magnetic field may be obtained by rotating the magnet.

In some examples of the magnetomotive imaging probe, the magnet may be a diametral magnet.

In some examples of the magnetomotive imaging probe, the ultrasound transducer may be arranged along a length of the magnet. Preferably, a length of the cylindrical magnet exceeds a width of the ultrasound traducer.

In some examples of the magnetomotive imaging probe, a distance between the magnet and the sensing device may be defined mainly by the thickness of the housing at the position of the sensing device and/or a distance between an inner surface of the housing and the magnet.

In some examples of the magnetomotive imaging probe, the magnet may be rotated using a motor.

In some examples of the magnetomotive imaging probe, the housing may have an elongated shape for positioning in a cavity of an animal, such as a human. The device may be configured for transrectal or transvaginal use.

In some examples of the magnetomotive imaging probe, the housing may be made of a non-electrically conductive material.

In some examples of the magnetomotive imaging probe, the ultrasound transducer may be an array, such as a linear array or a 2D array.

In some examples of the magnetomotive imaging probe, the array may be arranged concentrically with the magnet.

In some examples of the magnetomotive imaging probe, the housing may have a probe portion and a handle portion. The magnet and sensing device may be arranged at a distal portion of the probe portion and the motor may be arranged in the handle portion and may rotate the magnet via a shaft extending there between.

In some examples of the magnetomotive imaging probe, a sensor may be used for tracking the position of the magnet during the rotation. Other examples of sensors that may be used are sensors to detect probe position, temperature, acceleration or other physical parameters.

In some examples of the magnetomotive imaging probe, a disk may be arranged at each side of the magnet. Each disk may have a peg arranged in the center of each disk for holding the magnet. And one of the pegs may be configured for connecting to the motor or the shaft connected to the motor.

In some examples of the magnetomotive imaging probe, a packing, such as an O-ring, may be arranged at each side of the magnet for centering the magnet by use of the pegs.

Some advantages with the described device are that the imaging and analysis of tissue of humans and large animals are made possible at more locations compared to the previous techniques. This without the most hampering drawback with the prior techniques which is that valuable magnetic field strength close to the ultrasound transducer is lost due to the separation of ultrasound transducer and magnet which improves the sensitivity of detection of magnetic nanoparticles.

Further embodiments of the invention are defined in the dependent claims, wherein features for the second and subsequent aspects of the invention are as for the first aspect mutatis mutandis.

It should be emphasized that the term "comprises/comprising" when used in this specification is taken to specify the presence of stated features, integers, steps or components but does not preclude the presence or addition of one or more other features, integers, steps, components or groups thereof.

### Brief Description of the Drawings

These and other aspects, features and advantages of which embodiments of the invention are capable of will be apparent and elucidated from the following description of embodiments of the present invention, reference being made to the accompanying drawings, in which
Figs. 1A and 1B are illustrating a schematic a magnetomotive imaging probe assembly according to an example of the present disclosure;
Figs. 2A to 2E are schematic illustrations of different exemplary ultrasound transducer configurations at a distal portion of the probe;
Fig. 3 is illustrating a schematic example of an outer housing of the probe according to the disclosure;
Fig. 4 is illustrating a schematic example of cross-section of the probe illustrated in Fig. 3;
Fig. 5A and 5B are illustrating a schematic example of the magnet and ultrasound arrangement according to the disclosure; and
Fig. 6 is illustrating a flowchart of an exemplary method according to the disclosure.

### Description of examples

Specific examples of the disclosure will now be described with reference to the accompanying drawings. This disclosure may, however, be embodied in many different forms and should not be construed as limited to the examples set forth herein; rather, these examples are provided so that this disclosure will be thorough and complete, and will fully convey the scope of the disclosure to those skilled in the art. The terminology used in the detailed description of the examples illustrated in the accompanying drawings is not intended to be limiting of the disclosure. In the drawings, like numbers refer to like elements.

The following description focuses on an example of the present disclosure applicable to a probe assembly for magnetomotive imaging. However, it will be appreciated that the disclosure is not limited to this application but may be applied to many other fields and applications.

In magnetomotive imaging, a magnet is used to generate an inhomogeneous magnetic field. The inhomogeneous magnetic field may be a time-varying magnetic field at the same location as an imaging plane of an ultrasound transducer wherever the probe is positioned spatially relative the imaged subject. Magnetic nanoparticles, for example made from magnetite (Fe₃O₄), maghemite or zinc-doped magnetite, and usually with a size range of 10-100 nm (incl coating), are located at the target location within the imaging plane.

The time-varying magnetic field (T) is provided at a target location in the imaging plane of the ultrasonic transducer. Magnetic nanoparticles that are located at the target location in the imaging plane thus exhibit spatial fluctuations in the time-varying magnetic field (T) and are therefore forced to translate, displace, and/or rotate under the influence of the magnetic field (T). Because of the creation of the time-varying magnetic field component in the axial direction of the imaging plane, the displacement amplitude of the nanoparticles and their surrounding can be detected by the ultrasonic transducer. The time varying magnetic field may have a gradient which may cause the nanoparticles to translate, displace and/or rotate under the influence of the magnetic field (T). Therefore, the translatory or displacement motion of the particles may be oscillating due to the time variation of the magnetic field.

The shape of the particle may further enhance or suppress the action of translation, displacement, and/or rotation. For example, particles having an oval/elliptic shape may give rise to a rotational movement which together with a translational movement may be detectable with the ultrasound transducer and could be used to improve the detection. Spherical particles may also rotate due to the gradient of the time-varying magnetic field. Further, other shapes, such as rod shaped, octagonal or star shaped particles may also give rise to movements which may be used to improve the detection.

By observing the movement and displacement of the nanoparticles in the imaging plane an accurate representation of the concentration can be found which could be used to outline an object in the magnetomotive image or spots in a tissue with increased nanoparticle concentration.

The displacement of the nanoparticles may also be dependent on the distance between the magnet and where in the imaging plane the nanoparticles are positioned.

Fig. 1A is illustrating a schematic magnetomotive imaging probe device 100. The device includes a housing 101 having an outer surface and an inner cavity 107. The device may further include a magnet 102 arranged in the inner cavity 107 of the housing 101. The magnet 102 is configured for generating a time-varying magnetic field. The magnet 102 may be a cylindrically shaped permanent magnet, preferably a diametrically magnetized magnet. Such a diametral magnet is a cylindrically shaped magnet, having a rotational axis extending in a lateral direction. This type of magnet provides a magnetic field orthogonally to the axis. In some example may the shape of the magnet be a rectangular cuboid, such as a cube, rotating around an axis and provides a magnetic field orthogonally to the axis, similar as for a diametral magnet. The magnet has opposite magnetic poles (N, S) separated along a diameter of the magnet in the radial direction (r), whereby rotation of the magnet, create a time-varying magnetic field (T), at a target location. It is conceivable that other shapes of the magnet 102, may provide the same effect.

The time-varying magnetic field may be obtained by rotating the magnet 102 using a motor 104. The magnet 102 may be rotated by the motor 104 via a shaft 105. Alternatively, in some examples may an electric magnet be used, and the time-varying magnetic field may be obtained by switching the poles, driving a sinusoidal current, or any time-varying current that results in the desired magnetic field.

For detecting the translation displacement and/or rotation of the particles, a distance, movement, or magnetic material sensing device 103, for example an ultrasound transducer, such as an ultrasound array, an optical sensor (for example laser Doppler), a susceptometer, or other means for detecting distance, movement, or magnetic material known to the person skilled in the art, may be used. For the rest of this application, an ultrasound transducer will be used as an example of a sensing device, but as described other types of sensing devices could be used instead.

The ultrasound transduced may be arranged on the outer surface of the housing 101 so that an imaging plane of the ultrasound transducer 103 is within the time-varying magnetic field. In particular, this may be achieved by arranging an ultrasound transducer 103 on an outer surface of the housing 101 so that the ultrasound transducer 103 is positioned along a length of the magnet 102. Preferably a length of the cylindrical magnet 102 exceeds a width of the ultrasound transducer 103. The ultrasound transducer 103 will then be positioned within the time varying magnetic field together with the imaging plane of the ultrasound transducer 103. This arrangement may increase the sensitivity of detection of magnetic nanoparticles inside the volume of the magnetic field moving due to the variation of the magnetic field.

Typically, ultrasound pulses in the geometric shape of a beam, are fired using a number of the elements in the array, whereby a focused beam may be achieved by delaying the transmission of certain elements such that the sound interferes constructively at the focus. Likewise, the returning echoes may be detected on a number of elements, and a reconstructed signal may be achieved such that echoes along the transmit beam are amplified, by the same delays as in the transmit case. Typically, the magnetic particles are much smaller than the resolution cell of the ultrasound, but their displacement upon the action of the magnetic field, may create a detectable motion in their immediate surrounding, such that the motion can be detected by ultrasound. This can be done by covering the imaging plane by imaging beams at a rate exceeding that of the magnetic field frequency. Theoretically the rate of imaging (probing) at one location must be at least twice that of the induced motion of the nanoparticles, but practically even higher, 4-10 times for instance. Other imaging schemes are possible, such as creating a plane wave insonifying a large portion of the region to be imaged.

The sensitivity may be further enhanced by arranging the magnet 102 and the ultrasound transducer 103, in such a way that the active ultrasound elements are situated as close as practically possible to the magnet 102. When the ultrasound transducer is arranged on the housing, as illustrated in Figs. 1A and 1B, a distance between the magnet 102 and the ultrasound transducer 103 may be defined mainly by the thickness of the material of housing 101 at the position of the ultrasound transducer and/or a distance between an inner surface of the housing and the magnet 102.

Fig. 1A shows the case for a rotating permanent magnet 102, magnetized diametrically, whereby a multitude of ultrasound elements are arranged in an arc that is concentric with the rotation axis of the magnet 102. This concentric arrangement is also illustrated in Fig. 1B. Upon rotation of the magnet 102, the magnetic field at a point some distance from the probe 100, may also rotate and induce a rotating motion to magnetic particles located at that point. In Figs. 1A and 1B, the imaging plane is orthogonal to the rotation axis 108 of the magnet 102.

The probe 100 may further include connectors 106, such as cables, for controlling the probe and for transferring recorded data, such as imaging data from the ultrasound transducer 103 to a computer or a control unit which includes a processor. Additionally, and/or alternatively, the probe may be controlled, and the data may be transferred wirelessly, such as over Wi-Fi or other types of wireless protocols, such as Bluetooth.

Additionally, and/or alternatively, the cables 106 may be used for providing power to the probe. Alternatively, the power to the probe may be provided using batteries arranged in the probe.

The motor 104 of the magnetomotive imaging probe device 100 may be controlled by a control unit (not shown). As described above, the motor 104 may be coupled to the control unit via the connectors 106 or through a wireless protocol. The power of the motion of the magnet 102 may then be controlled. The control unit may be adapted to vary the speed of motion (w) of the magnet 102, according to a predetermined pattern to thereby vary the frequency of the time-varying magnetic field (T) as a predetermined impulse to generate an impulse response of magnetic nanoparticles at the target location. This provides for determining an impulse response from the nanoparticles that may be indicative of the material properties, such as viscosity and density. Hence the nanoparticles may be displaced by the magnetic impulse and the material properties will affect how the displacement varies over time, such as the dominant frequency, maximum amplitude, and speed of damping may be indicative of material density, the elasticity and viscosity.

The control unit may be adapted to vary the speed of motion (w) of the magnet 102, such as by increasing the speed linearly up to a certain maximum speed, and thereafter decrease the speed, to provide a sweep throughout frequencies (chirp) and detect the resulting displacement amplitude of the nanoparticles. The control unit may thus be adapted to vary the speed of motion (w) of the magnet 102, according to such predetermined pattern to provide for detection of a frequency impulse response. The control unit may be adapted to set a constant speed of motion (w) of the magnet 102. Depending on the position of the S-pole relative the N-pole during rotation of the magnet the rotational force may vary, for instance due to influence of the earth magnetic field or iron structures in the proximity, which thus may be compensated by the control unit.

The control unit may be further adapted to synchronize the frequency or speed of motion (w) of the magnet 102, to the ultrasound imaging in order to provide for ultrasound detection at the magnetic field frequency (that of the magnetic field time-variation), and further to allow for detection at the phase of the magnetic field relative the ultrasound imaging.

The ultrasound transducer 103 may also have an ultrasound control unit (not shown) that provides for the necessary control and analysis related to the ultrasound equipment.

The device may include different sensors, for example a sensor for for tracking the position of the magnet during the rotation. This could be an encoder for detecting a pulse on each rotation. This may be useful for synchronize the frequency or speed of motion (w) of the magnet 102, to the ultrasound imaging or when having a gearbox, such as reduction gear. Other sensors that could be used are, an accelerometer to detect if the device is held steady or is moving, a gyroscope, thermometer, a pH sensor, a sensor for detecting interfering magnet fields.

The housing should be made of a non-electrically conductive material to avoid eddy currents which may reduce the delivered magnetic field. One such material is Polyether ether ketone (PEEK). Other materials could be Polyphenylene sulfide (PPS), or polysulfone.

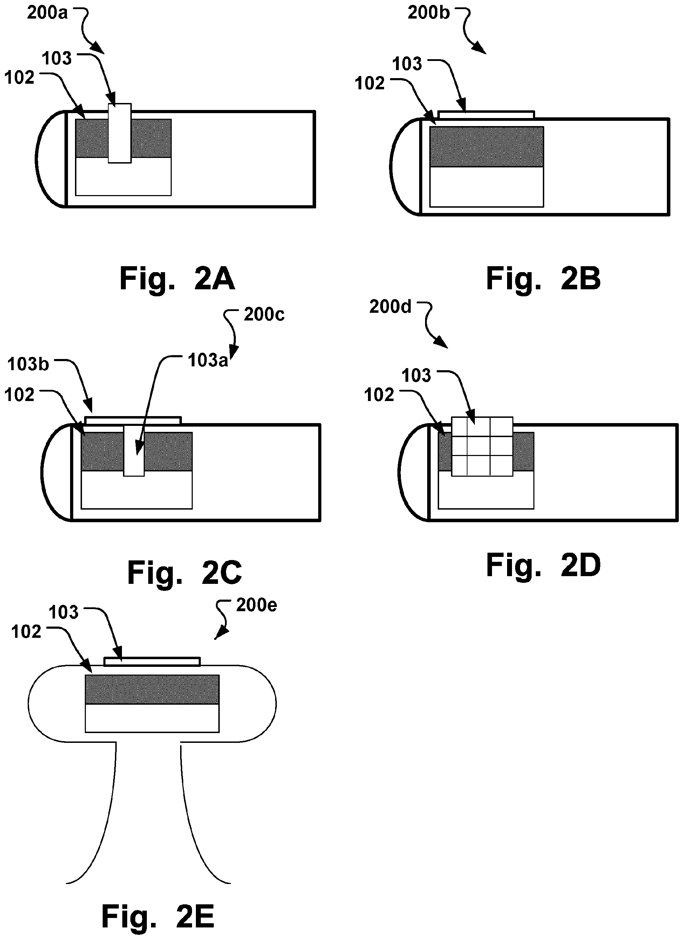

Figs. 2A to 2E are schematic illustrations of different exemplary ultrasound transducer configurations in relation to the magnet at a distal portion of the probe device. The transducer is in these examples different types of arrays. An array could be a single array with a number of ultrasound elements located in a line. The number of elements could be, as an example, between 128 to 256 depending on the size of the elements and the length of the array. A lower or higher number of elements may be used.

Fig. 2A is illustrating schematically an ultrasound array 103 arranged similar as in Figs 1A and 1B wherein the ultrasound array 103 is concentrically arranged with an axis of the magnet 102. As an example, for an array 103 that has between 128 and 256 elements and a central frequency of about 10 MHz, this arrangement may cover approximately 160-230 degrees field of view. As a further example, if the array 103 has 192 elements, this arrangement may cover approximately 180 degrees field of view, given an ultrasound center frequency of 7 to 8 MHz. The field of view may depend on the dimensions of the probe and the estimates given are for a probe to be used for transrectal or transvaginal applications.

Fig. 2B is illustrating schematically an ultrasound array 103 arranged orthogonally compared to the configuration illustrated in Fig. 2A. This means that the length of the array 103 is arranged along the length of the magnet 102.

Fig. 2C is illustrating schematically a combination of at least two arrays wherein a first ultrasound array 103a is arranged as in Fig. 2A and a second ultrasound array 103b is arranged as in Fig. 2B.

Fig. 2D is illustrating schematically a 2D array. The 2D array 103 is arranged concentrically with an axis of the magnet 102 but will record an image in two dimensions similar as the use of two arrays 103a, 103b illustrated in Fig. 2C.

Fig. 2E is illustrating a different type of distal end of the probe. While Fig. 1A and 1B as well as Figs. 2A to 2D are illustrating elongated devices which may be used, mainly, in a cavity of an animal or a human, such as for transrectal or transvaginal use. The illustrated device may improve external usage, such as on skin examination, that is for a transcutaneous ultrasound examination. The ultrasound array 103 could be arranged as illustrated in any of Figs. 2A to 2D in relation to the magnet 102.

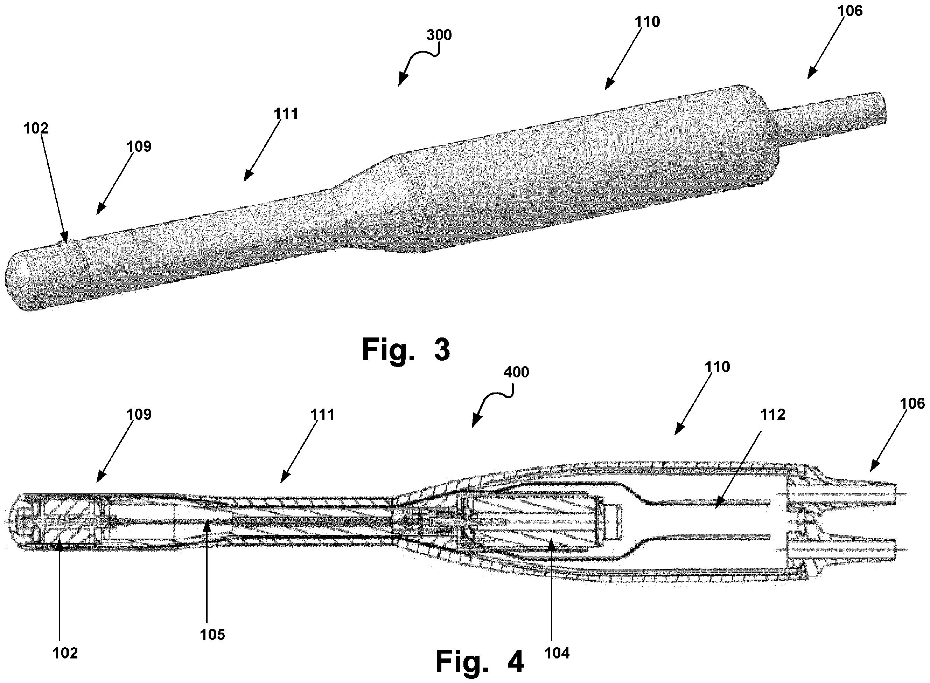

Fig. 3 is illustrating a schematic example of an outer housing 300 of the probe according to an example of the disclosure. The probe includes a distal portion 109 and a handle portion 110. At the distal portion 109, the ultrasound transducer 102 is arranged. The probe 300 may further include at least one connector 106 and/or a power cord. The distal portion 109 and the handle portion 110 may be connected via a neck portion 111. The neck portion 111 may have a smaller diameter compared to the distal portion 109. The probe 300 illustrated in Fig. 3 is made elongated and as smooth as possible as well as being dimensioned to be inserted into a cavity of a human or animal, such as transrectal or transvaginal. The device is not limited to be used in a cavity but could also be used externally.

To make the device comfortable, the distal portion 109 and the neck portion 111 have to be made "slim". Slim depends on the cavity but or transrectal use and for transvaginal use, the diameter should preferably be less than 30mm, such as 25mm, such as less than 20mm, such as about 15mm.

To further improve the positioning of the device in a cavity, a disposable sleeve may be arranged over the distal portion 109 and the neck portion 111 before being inserted into the cavity.

Alternatively, the distal portion may have a different shape for external use, such as having a broader tip to improve handling of the device, see for example Fig. 2E.

Fig. 4 is illustrating a schematic example of cross-section 400 of the probe illustrated in Fig. 3. As illustrated, the magnet 102 and the also the ultrasound transducer 109 is positioned as close as possible to the distal end of the distal portion 109. The motor 104 is positioned in the handle portion 110 and the shaft 105 connecting the magnet 102 and the motor 104 is arranged through the neck portion 111. For connecting and controlling the ultrasound transducer 102, a printed circuit board 112 is running through the device.

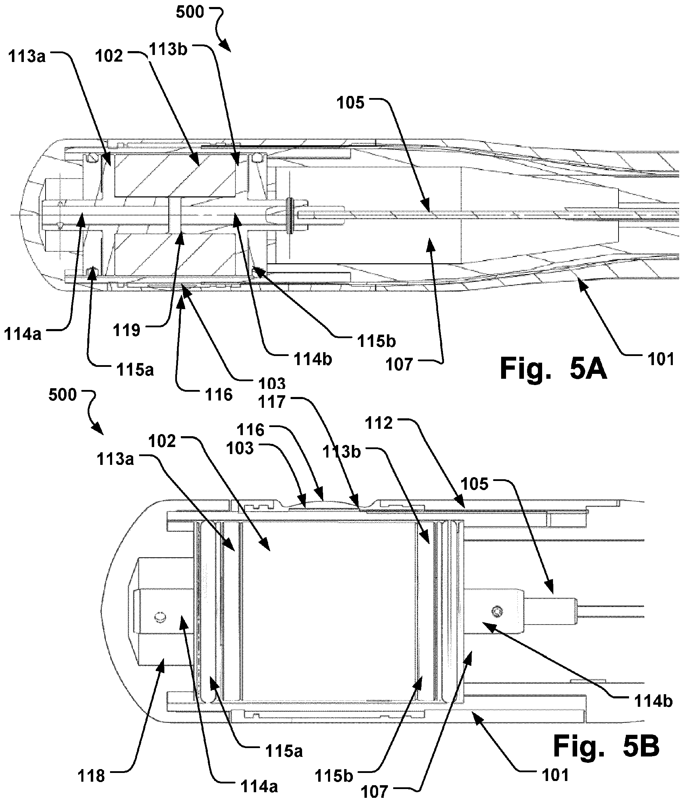

Figs. 5A and 5B are illustrating a schematic example 500 of the magnet and ultrasound arrangement according to the disclosure, Figs. 5A and 5B are illustrating cross-sectional views of an example of the ultrasound and magnet configuration.

The magnet 102 is arranged in the cavity 107 of the distal portion of the probe device. The cavity 107 is in the illustrated example a lumen which runs through the housing 101. On each side of the magnet 102 a disk 113a, 113b is arranged. The disk is preferably permeable to the magnetic field. Each disk 113a, 113b has a peg 114a, 114b. The disks 113a, 113b and the pegs 114a, 114b are used to hold the device. the pegs 114a, 114b runs through a central lumen 119 of the magnet 102. To minimize the vibrations, the lumen 119 should run straight through the very center of the magnet 102. One way of achieving this with a low tolerance may be to arrange the disks 113a, 113b, with the pegs 114a, 114b, on the magnet 102 before the magnet is lathed into its shape. By holding the magnet by the disks 113a, 113b and the pages 114a, 114b during the lathing, a high accuracy can be obtained.

To further minimize any vibrations when rotating the magnet 102, a packing 115a, 115b, such as an O-ring, may be arranged at each side of the magnet 102 for centralizing the magnet 102 in the lumen 107 using the pegs 114a, 114b. the most distal peg 114a may be arranged in a holder 118 while the proximal peg 114b may be connected to the motor via shaft 105.

The ultrasound transducer 103 is arranged on an outer surface of the housing 101. In this way, the distance between the ultrasound transducer 102 and the magnet 102 may be minimized and is mainly composed of the thickness of the housing wall at the position between the magnet 102 and the ultrasound transducer 103. The distance may also be determined by a space between the magnet 102 and an inner surface of the housing 101. This distance may be determined by the tolerance required for rotating the magnet 102.

Since the ultrasound transducer 103 needs to be controlled, such as synchronization with the magnet, and transferring data to a control unit, such as a computer, etc. a printed circuit board, PCB, 112 may need to be connected to the ultrasound transducer 103. The ultrasound transducer is also arranged on a plate used for retaining the transducer to the housing. A notch 117may therefore be made outer surface of housing 101 surrounding magnet 102. In the notch 117, the components used for retaining the ultrasound transducer 103 as well as the connection to the PCB may be arranged, thus the part of these components which are arranged underneath the transducer, such as between the ultrasound transducer 103 and the magnet 102, may compose part of the thickness of the housing 101 at the location of the ultra sound transducer. The notch may therefore assist in minimizing the distance between the ultrasound transducer 103 and the magnet 102.

In total, the distance between the ultrasound transducer 103 and the magnet 102 may be less than 2mm, such as less than 1.5mm such as 1mm.

Additionally, the ultrasound transducer 116 may have a lens arranged on top for focusing the waves.

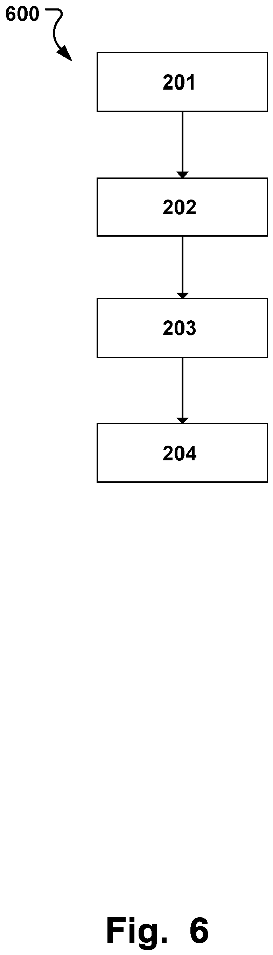

Fig. 6 is illustrating a flowchart of an exemplary method 200. The method 200 may include rotating 201 the magnet 102, to generate a time-varying magnetic field (T) at an imaging plane of the ultrasound transducer 103. The method further comprises detecting 204 motion of magnetic nanoparticles in response to the time-varying magnetic field with the ultrasound transducer 103 in the imaging plane. As mentioned above, this provides for an accurate determination of nanoparticle concentration, and further improved analysis of the examined material. The method 200 may further include rotating 202 the cylindrical permanent magnet 102 according to a predetermined pattern to thereby vary the frequency of said time-varying magnetic field (T) as a predetermined frequency impulse to generate a frequency impulse response of the magnetic nanoparticles. The properties of the material of the analyzed object may thus me determined. The predetermined pattern may for example include rotating the magnet with a certain number of turns, or fractions of turns, such as half a turn, during a period of time such as a certain number, or fractions of seconds or minutes, to subsequently detect the response from the nanoparticles.

The method 200 may alternatively or in addition comprise rotating 203 first 102 and second 103 cylindrical permanent magnet with a constant rotational speed.

As will be appreciated by one of skill in the art, the present invention may be embodied as device, system, or method.

The present disclosure has been described above with reference to specific embodiments. However, other examples than the above described are equally possible within the scope of the invention. Different method steps than those described above, may be provided within the scope of the invention. The different features and steps of the invention may be combined in other combinations than those described. The scope of the disclosure is only limited by the appended patent claims.

More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

## Claims

1. A magnetomotive imaging probe device, comprising:
a housing having an outer surface and an inner cavity;
a magnet;
a sensing device configured to detect distance, movement, or magnetic material; and
wherein the magnet is arranged in the inner cavity of the housing and the sensing device on the outer surface of the housing, and the magnet is arranged to generate a time-varying magnetic field at an imaging plane of the sensing device.

2. The magnetomotive imaging probe of claim 1, wherein the sensing device is an ultrasound transducer.

3. The magnetomotive imaging probe of any of claims 1 or 2, wherein the magnet is a cylindrically shaped permanent magnet and wherein the time-varying magnetic field is obtained by rotating the magnet.

4. The magnetomotive imaging probe of claim 3, wherein the magnet is a diametral magnet.

5. The magnetomotive imaging probe of any of claims 2 to 4, wherein the ultrasound transducer is arranged along a length of the magnet, preferably a length of the cylindrical magnet exceeds a width of the ultrasound traducer.

6. The magnetomotive imaging probe of any of claims 1 to 5, wherein a distance between the magnet and the sensing device is defined mainly by the thickness of the housing at the position of the sensing device and/or a distance between an inner surface of the housing and the magnet.

7. The magnetomotive imaging probe of any of claims 3 to 6, wherein the magnet is rotated using a motor.

8. The magnetomotive imaging probe of any of claims 1 to 7, wherein the housing has an elongated shape for positioning in a cavity of an animal, such as a human, such as the device is configured for transrectal or transvaginal use.

9. The magnetomotive imaging probe of any of claims 1 to 8, wherein the housing is made of a non-electrically conductive material.

10. The magnetomotive imaging probe of any of claims 2 to 9, wherein the ultrasound transducer is an array, such as a linear array or a 2D array.

11. The magnetomotive imaging probe of any of claim 10, wherein the array is arranged concentrically with the magnet.

12. The magnetomotive imaging probe of any of claims 7 to 11, wherein the housing has a probe portion and a handle portion, wherein the magnet and sensing device is arranged at a distal portion of the probe portion and the motor is arranged in the handle portion and rotates the magnet via a shaft.

13. The magnetomotive imaging probe of any of claims 2 to 12, wherein a sensor is used for tracking the position of the magnet during the rotation.

14. The magnetomotive imaging probe of any of claims 2 to 13, wherein a disk is arranged at each side of the magnet, each disk has a peg arranged in the center of each disk for holding the magnet.

15. The magnetomotive imaging probe of claim 14, wherein a packing, such as an O-ring, is arranged at each side of the magnet for centering the magnet by use of the pegs.
